**Europäisches Patentamt**

**European Patent Office**

**Office européen des brevets**

(19)

(11) Veröffentlichungsnummer: **0 002 696**
**B1**

(12) **EUROPÄISCHE PATENTSCHRIFT**

(45) Veröffentlichungstag der Patentschrift: **22.04.81**

(21) Anmeldenummer: **78101594.6**

(22) Anmeldetag: **07.12.78**

(51) Int. Cl.³: **C 07 C 51/235,**
**C 07 C 51/16,**
**C 07 C 53/08, C 07 C 53/12**

(54) Verfahren zur Herstellung von Essigsäureanhydrid und Essigsäure aus Acetaldehyd.

(30) Priorität: **22.12.77 DE 2757222**

(43) Veröffentlichungstag der Anmeldung:
**11.07.79 Patentblatt 79/14**

(45) Bekanntmachung des Hinweises auf die Patenterteilung:
**22.04.81 Patentblatt 81/16**

(84) Benannte Vertragsstaaten:
**BE DE FR GB IT NL**

(56) Entgegenhaltungen:
**US - A - 2 658 914**

(73) Patentinhaber: **HOECHST AKTIENGESELLSCHAFT**
**Postfach 80 03 20**
**D-6230 Frankfurt/Main 80 (DE)**

(72) Erfinder: **Erpenbach, Heinz, Dr.**
**Oberbuschweg 22**
**D-5000 Köln 50 (DE)**
Erfinder: **Gehrmann, Klaus, Dr.**
**Geschwister-Scholl-Strasse 43**
**D-5042 Erftstadt (DE)**
Erfinder: **Hauser, Alfred, Dr.**
**Nikolaus-Ehlen-Strasse 47**
**D-5042 Erftstad (DE)**
Erfinder: **Karrenbauer, Kurt, Dr.**
**Lange Heide 38**
**D-5042 Erftstadt (DE)**
Erfinder: **Lork, Winfried**
**Tulpenweg 1**
**D-5042 Erftstadt (DE)**

EP 0 002 696 B1

## Verfahren zur Herstellung von Essigsäureanhydrid und Essigsäure aus Acetaldehyd

Die Herstellung von Essigsäureanhydrid als Hauptprodukt und Essigsäure als Nebenprodukt durch Oxydation von Acetaldehyd in Gegenwart von Kupfer- und Cobaltacylat als Katalysator in der Flüssig- oder Gasphase ist bekannt (vgl. Stanford Research Institute, Menlo Park, California, USA, Report No. 37 A (März 1973), Seiten 171—179; Ullmann's Encyklopädie der technischen Chemie, 4. Aufl. Band 11 (1976), Seiten 81—82; DE—PS 1 192 637).

In der flüssigen Phase wird zur Oxydation des Acetaldehyds im Gemisch mit Äthylacetat und Essigsäure bei 40—60°C reiner Sauerstoff eingesetzt. Die Reaktionsprodukte werden in flüssigem Zustand gemeinsam mit dem Verdünnungsmittel und dem Katalysator aus dem Reaktor abgezogen. Verdünnungsmittel, Katalysator und nichtumgesetzter Acetaldehyd werden zum Reaktor zurückgeführt. Optimale Essigsäureanhydrid-Ausbeuten von 70 Mol% werden im Flüssigphase-Verfahren nach dem bekannten Stand der Technik nur dann erhalten, wenn nicht mehr als 70 bis 80% des angebotenen Acetaldehyds umgesetzt werden und das Gewichtsverhältnis Äthylacetat zu Acetaldehyd = 70 : 30 im Einsatzgemisch beträgt. Im Gasphase-Verfahren werden demgegenüber bei 60°C Acetaldehyd und Luft dem Reaktor zugeführt, der eine Mischung aus Essigsäureanhydrid, Essigsäure, Hochsiedern und Katalysator enthält und in dem eine Konzentration von 4—6 Gew% Acetaldehyd eingestellt ist. Die Reaktionsprodukte werden mit nichtumgesetztem Acetaldehyd und überschussiger Luft gasförmig aus dem Reaktor abgezogen. Acetaldehyd sowie die Hauptmenge der Luft werden zum Reaktor zurückgeführt. Auch dieses Verfahren erlaubt nur einen partiellen Acetaldehydumsatz von 30—50%, um Essigsäureanhydrid-Ausbeuten von 70—80 Mol% zu gewährleisten.

Die Nachteile beider Verfahren sind offensichtlich. So besitzt das Flüssigphase-Verfahren zwar den Vorteil eines gegenüber dem Gasphase-Verfahren höheren Acetaldehydumsatzes, ist jedoch mit dem Nachteil behaftet, daß etwas mehr Essigsäure und entsprechend weniger Essigsäureanhydrid gebildet wird. Das Gasphase-Verfahren hat neben dem erheblich geringeren Acetaldehydumsatz darüber hinaus den Nachteil eines hohen, zur gasförmigen Austragung der Reaktionsprodukte erforderlichen, energieaufwendigen Gaskreislaufes. Für beide Verfahren ist jedoch der zum Erhalt hoher Essigsäureanhydrid-Ausbeuten erforderliche mehr oder weniger partielle Acetaldehydumsatz, der die Aufarbeitung und Rückführung größerer Mengen nichtumgesetzten Acetaldehyds bedingt, besonders nachteilig.

Die Erfindung betrifft nun ein Verfahren zur kontinuierlichen Herstellung von Essigsäure und Essigsäureanhydrid durch Oxydation von Acetaldehyd mit Sauerstoffgas in flüssiger Phase in Gegenwart von Kupferacylat und Kobaltacylat als Katalysator und in Gegenwart eines aliphatischen Carbonsäureesters als Verdünnungsmittel, welches dadurch gekennzeichnet ist, daß man die Umsetzung bei Temperaturen von 62 bis 90°C und Verweilzeiten von weniger als 20 min mit einem Gewichtsverhältnis der Verdünnungsmittels zu Acetaldehyd von 60 : 40 bis 40 : 60 durchführt, und daß man die Reaktionsprodukte einer Destillationszone zuführt, über Kopf Carbonsäureester und Wasser abdestilliert, in zwei Phasen trennt, die organische Phase unter einem Rückfluß von mindestens 1 : 1 zur Destillationszone zurückführt, das Sumpfprodukt der Destillationszone einer Verdampferzone zuleitet und dort 2 bis 12 Gewichtsteile eines Essigsäure/Essigsäureanhydrid-Gemisches als Destillat je 1 Gewichtsteil Katalysatorlösung als Sumpfprodukt gewinnt.

Darüberhinaus kann das Verfahren der Erfindung vorzugsweise dadurch gekennzeichnet sein, daß man

a) die Umsetzung in einer langgestreckten, senkrecht-stehenden Reaktionszone durchführt und die berechnete Menge Sauerstoffgas gleichzeitig an mehreren Stellen unterschiedlicher Höhe in die Reaktionszone eindosiert;

b) das Sauerstoffgas in einer solchen Menge in die Reaktionszone eindosiert, das der Sauerstoffgehalt des Abgases geringer als 5 Vol% ist.

Das Verfahren der Erfindung vermeidet die geschilderten Nachteile. Als Reaktionszone dient bevorzugt ein in der Technik gebräuchlicher Turmreaktor, in den der benötigte Sauerstoff über die Reaktionsstrecke verteilt eingeleitet wird. So erzielt man hohe Acetaldehydumsätze von über 95% und Essigsäureanhydrid-Ausbeuten, berechnet auf umgesetzten Acetaldehyd, von über 70 Mol%. Außerdem kann die Acetaldehydkonzentration in der Einsatzmischung beträchtlich höher liegen als gemäß dem Stand der Technik z.B. bei 40 Gew%.

Die Umsetzung gemäß der Erfindung wird zweckmäßig bei Drucken von 1—5 bar, vorzugsweise von 2,5—3 bar, durchgeführt. Dabei ist die Einhaltung von Verweilzeiten im Reaktor von 6 bis 12 min und Temperaturen von 65—70°C vorteilhaft. Die Kupfer- und Kobaltacylate liegen in der Reaktionsmischung bevorzugt in einem Gewichtsverhältnis, gerechnet als $CuO : Co_3O_4$, von 1 : 0,25—1 vor. Es ist günstig, als Acylate die Acetate, und zwar in einer Gesamtkonzentration von 0,08—0,2 Gew%, gerechnet als $CuO + Co_3O_4$ und bezogen auf die Reaktionsmischung, einzusetzen. Als Carbonsäureester finden bevorzugt Äthylacetat und Isopropylacetat bei einem Gewichtsverhältnis von Ester zu Acetaldehyd = 50 : 50 Verwendung.

Es ist weiterhin zweckmäßig, die organische Phase unter einem Rückfluß von 1,5 bis 2,5 : 1 zur Destillationskolonne zurückzuführen und im Verdampfer 6 bis 10 Gewichtsteile eines Gemisches aus Essigsäure und Essigsäureanhydrid als Destillat je 1 Gewichtsteil Katalysatorlösung als Sumpfprodukt, das in die Reaktionszone zurückgeführt wird, zu gewinnen.

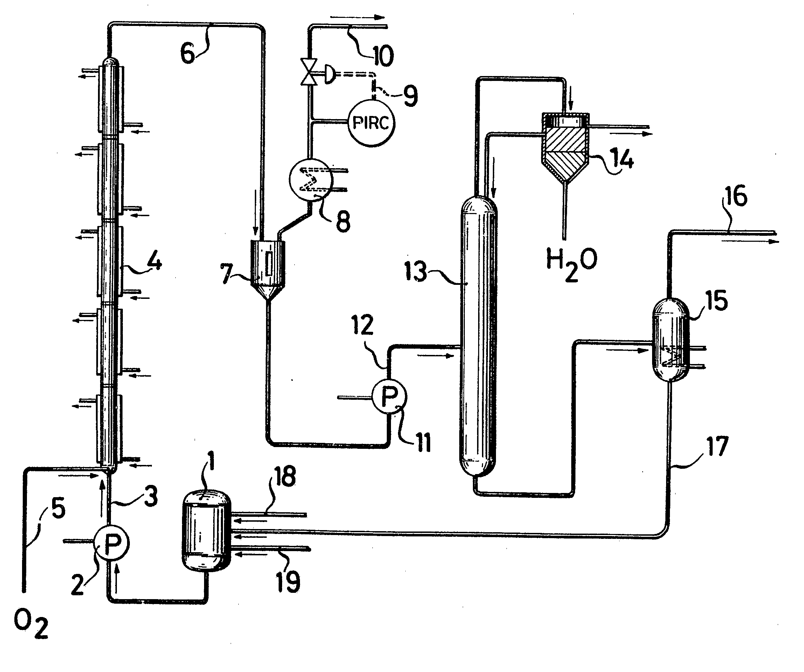

Eine der möglichen Vorrichtungen zur Druchführung des erfindungsgemäßen Verfahrens ist in der Zeichnung schematisch dargestellt und im folgenden beschrieben:

Aus dem Behälter (1) wird eine Mischung aus Acetaldehyd, Carbonsäureester, Essigsäure, Acetanhydrid und Katalysator über die Dosierpumpe (2) und Leitung (3) dem Boden des Reaktors (4) zugeführt. Gleichzeitig wird über Leitung (5) die zur Oxydation notwendige Menge Sauerstoff über drei in verschiedener Höhe angeordnete Einlaßstutzen dem unteren Teil des Reaktors (4) zudosiert. Der Reaktor (4) ist in 5 Segmente unterteilt, die jeweils zur Kühlung ummantelt sind. Jedes Segment hat seinen eigenen Kühlkreislauf. Auf diese Weise ist es möglich, die bei der stark exothermen Reaktion auftretenden Wärmemengen so absuführen, daß in jedem Segment ein etwa gleiches Temperaturprofil vorherrscht. Die Reaktionsprodukte strömen flüssig am Kopf des Reaktors (4) über Leitung (6) ab. Im Abscheider (7) trennen sich die geringen Mengen Abgas von der Flüssigkeit und verlassen über den Kühler (8), das Druckhalteventil (9) und die Leitung (10) die Apparatur. Die flüssigen Produkte werden unter Standhaltung im Abscheider (7) mittels der Dosierpumpe (11) über Leitung (12) zur Destillations-Kolonne (13) abgezogen. Kolonne (13) besteht aus 40 Glockenböden, der Einlauf befindet sich in der Mitte. Über Kopf der Kolonne (13) fallen Carbonsäureester, nichtumgesetzter Acetaldehyd und Reaktionswasser als Destillat an. Nach Phasentrennung im Abscheider (14) wird mindestens 1 Teil je Teil abgenommener organischer Phase als Rückfluß der Kolonne (13) wieder aufgegeben. Das Sumpfprodukt der Kolonne (13) wird dem Verdampfer (15) zugeleitet, wo produzierte Essigsäure und Acetanhydrid als Destillat über Leitung (16) gewonnen werden. Das den Katalysator, gelöst in einer Mischung aus Essigsäure, Acetanhydrid und geringen Mengen Hochsieder, enthaltende Sumpfprodukt wird über Leitung (17), der nichtumgesetzte Acetaldehyd wird über Leitung (18) und der entwässerte Carbonsäureester wird über Leitung (19) zum Behälter (1) zurückgeführt.

Die Erfindung wird durch folgende Beispiele näher erläutert:

Beispiel 1

Die Ausgansmischung enthält 40 Gew% Acetaldehyd, 40 Gew% Äthylacetat, 3,7 Gew% Acetanhydrid, 14,1 Gew% Essigsäure, 1,93 Gew% Hochsieder und 0,27 Gew% Katalysator (0,18 Gew% Cu-acetat + 0,09 Gew% Co-acetat) mit einem Gewichtsverhältnis der Oxide $CuO : Co_3O_4 = 1 : 0,5$ (= 0,08 Gew% CuO + 0,04 Gew% $Co_3O_4$). Stündlich werden 4,27 kg (4,8 1) Ausgangsmischung, die 1708 g Acetaldehyd (38,8 Mol) sowie 160 g Acetahnydrid und 602 g Essigsäure enthalten, am Boden des Reaktors (4) aufgegeben. Der Reaktor besteht aus 5 ummantelten Segmenten von je 0,5 m Länge und 24 mm Durchmesser und ist mit Füllkörpern gefüllt. Gleichzeitig mit der Ausgangsmischung werden insgesamt 450 l/h $O_2$ (20 Mol), im Verhältnis 2 : 1 : 1 aufgeteilt, am Fuße des ersten, zweiten und dritten Segmentes zudosiert. Die $O_2$-Menge ist dabei so bemessen, daß ein $O_2$-Wert von 5 Vol% im Reaktorabgas nicht überschritten wird. Die Temperatur der einzelnen Segmente wird durch indirekte Kühlung mit Wasser konstant gehalten. Die Temperaturmessung im Reaktor erfolgt mittels in einem Thermorohr befindlicher Thermoelemente. Die Reaktionstemperatur beträgt 75°C, der Druck 2,8 bar. Bei einem Reaktorvolumen von 800 ml unter Betriebsbedingungen ergibt sich somit eine Verweilzeit von 10 min. Stündlich werden über Kopf des Reaktors 4,9 kg flüssiges Reaktionsprodukt abgezogen und dem 20. Boden der Destillationskolonne (13) mit insgesamt 40 Böden und 80 mm Durchmesser aufgegeben. Bei 70°C Kopf- und 128°C Sumpftemperatur fallen bei einem Rückfluß von 1,8 1,92 kg Esterphase mit 3 Gew% Wasser und 2,6 Gew% Acetaldehyd (50 g) sowie 0,21 kg wässerige Phase an. 2,77 kg/h Sumpfprodukt gelangen in den Verdampfer (15) und werden bei 83°C und 173 mbar im Verhältnis 6 Gewichtsteile Destillat zu 1 Gewichtsteil Katalysatorlösung als Sumpfablauf zerlegt. So werden einerseits 2,37 kg/h Destillat mit 58,2 Gew% Acetanhydrid (1379 g) und 41,8 Gew% Essigsäure (991 g) sowie andererseits 0,4 kg/h Katalysatorlösung mit 40 Gew% Acetanhydrid (160 g) und 36,8 Gew% Essigsäure (147 g) erhalten. Vom eingesetzten Acetaldehyd sind 96,9% (37,6 Mol) umgesetzt worden. Nach Abzug der mit der Ausgangsmischung eingesetzten (nicht produzierten) Mengen Essigsäure und Acetanhydrid betragen, bezogen auf die umgesetzte Menge Acetaldehyd, die Ausbeuten an Acetanhydrid 1379 g/h (13,52 Mol) = 71,9% und an Essigsäure 536 g/h (8,93 Mol) = 23,8%. Die Gesamtausbeute an Acetanhydrid und Essigsäure beläuft sich demnach auf 95,7%. Die Raum-Leistung beträgt 1741 g Acetanhydrid + Essigsäure je Liter freies Reaktorvolumen und Stunde. Die Katalysatorlösung sowie die Anteile nicht produzierte Essigsäure und Acetanhydrid werden gemeinsam mit den geringen Mengen nicht umgesetzten Acetaldehyds und der von Leichtsiedern und Wasser befreiten Esterphase zum Ansatzgefäß (1) zurückgeführt.

### Beispiel 2

Es wird wie in Beispiel 1 gearbeitet mit folgenden Abweichungen:

Stündlich werden 6,1 kg (6,85 l) Ausgangsmischung in der Zusammensetzung 40 Gew% Acetaldehyd (55,5 Mol), 15,9 Gew% Essigsäure (970 g), 2,5 Gew% Acetanhydrid (153 g), 40 Gew% Äthylacetat, 1,28 Gew% Hochsieder und 0,32 Gew% Katalysator (Cu-acetat + Co-acetat) mit einem Gewichtsverhältnis der Oxide $CuO : Co_3O_4 = 1 : 0,5$ (= 0,093 Gew% CuO + 0,047 Gew% $Co_3O_4$) dem Reaktor (4) zugeführt. 640 l/h $O_2$ werden, im Verhältnis 3 : 2 : 1 aufgeteilt, am Fuße des 1., 2. und 3. Segmentes aufgegeben. Dabei wird ein $O_2$-Wert im Reaktorabgas von kleiner als 5 Vol% eingehalten. Die Reaktionstemperatur beträgt 75°C, der Druck 2,8 bar. Die Verweilzeit beläuft sich auf 7 min. Über Kopf des Reaktors fließen stündlich 6,9 kg Reaktionsprodukt ab. Der Acetaldehydumsatz beträgt 96%. Under einem Rückfluß von 2 werden in der Destillationskolonne (13) 2,7 kg/h Esterphase mit 3 Gew% Wasser und 3,6 Gew% Acetaldehyd (97 g) sowie 0,28 kg/h wässerige Phase abgetrennt. 3,92 kg/h Sumpfprodukt gelangen in den Verdampfer (15) und werden im Verhältnis 10 Gewichtsteile Destillat zu 1 Gewichtsteil Katalysatorlösung als Sumpfablauf zerlegt. So werden 3,56 kg/h Destillat mit 57,9 Gew% Acetanhydrid (2060 g) und 42,1 Gew% Essigsäure (1500 g) sowie 0,36 kg/h Katalysatorlösung mit 42,5 Gew% Acetanhydrid (153 g) und 30,5 Gew% Essigsäure (110 g) gewonnen. Nach Abzug der mit der Ausgangsmischung eingesetzten Mengen betragen, bezogen auf den Acetaldehydumsatz, die Ausbeuten an Acetanhydrid 2060 g/h (20,2 Mol) = 75,8% und an Essigsäure 640 g/h (10,7 Mol) = 20%. Die Gesamtausbeute beläuft sich demnach auf 95,8%. Die Raumleistung beträgt 2455 g Acetanhydrid + Essigsäure je Liter freies Reaktorvolumen und Stunde.

### Beispiel 3

Es gelten die Bedingungen des Beispiels 1, wobei anstelle von Äthylacetat Isopropylacetat als Verdünnungsmittel eingesetzt wird. Stündlich werden 4.27 kg Ausgangsmischung mit 40 Gew% Acetaldehyd (38,8 Mol), 40 Gew% Isopropylacetat, 15,6 Gew% Essigsäure (666 g) und 2,6 Gew% Acetanhydrid (111 g) sowie den Katalysatormengen aus Beispiel 1 dem Reaktor (4) zugeführt. Gleichzeitig strömen über drei Stellen verteilt insgesamt 460 l/h $O_2$ ein. 4,84 kg/h Reaktionsprodukt gelangen in die Destillationskolonne (13). Unter einem Rückfluß von 1,5 fallen 1,9 kg/h Esterphase mit 2,8 Gew% Wasser und 2,9 Gew% Acetaldehyd (55 g) sowie 0,2 kg/h wässerige Phase an. Aus diesen Werten errechnet sich ein Acetaldehydumsatz von 96,8%. Bei einem Destillat-Sumpf-Gewichtsverhältnis von 6 : 1 werden im Verdampfer (15) 2,36 kg/h Destillat mit 58,1 Gew% Acetanhydrid (1371 g) und 41,9 Gew% Essigsäure (989 g) sowie 0,38 kg/h Katalysatorlösung mit 29,2 Gew% Acetanhydrid (111 g) und 55,2 Gew% Essigsäure (210 g) gewonnen. Nach Abzug der Einsatzmengen betragen, bezogen auf den Acetaldehydumsatz, die Ausbeuten an Acetanhydrid 1371 g/h (13,44 Mol) = 71,6% und an Essigsäure 533 g/h (8,9 Mol) = 23,7%. Die Gesamtausbeute beläuft sich auf 95,3%. Die Raumleistung beträgt 1731 g Acetanhydrid + Essigsäure/l.h.

### Beispiel 4 (Vergleichsbeispiel)

Es wird unter den im Beispiel 1 angegebenen Bedingungen gearbeitet, jedoch ist die Reaktionstemperatur auf 50°C gesenkt worden. Eingesetzt werden auch hier 4,27 kg/h Ausgangsmischung mit 40 Gew% Acetaldehyd (38,8 Mol), 15,6 Gew% Essigsäure (664 g) und 2,7 Gew% Acetanhydrid (117 g). Zur Einhaltung eines Sauerstoffspiegels von unter 5 Vol% im Reaktorabgas werden 340 l/h $O_2$ benötigt. 4,65 kg/h Reaktionsprodukt gelangen in die Destillationskolonne (13). Es werden 2,3 kg/h Esterphase mit 3,6 Gew% Wasser und 22,3 Gew% Acetaldehyd (512 g) sowie 0,1 kg/h wäßrige Phase erhalten. Daraus ergibt sich ein Acetaldehydumsatz von nur noch 70,1%. Bei einem Destillat-Sumpf-Gewichts-Verhältnis von 6 : 1 werden im Verdampfer (15), 1,93 kg/h Destillat mit 51,9 Gew% Acetanhydrid (1002 g) und 48,1 Gew% Essigsäure (928 g) sowie 0,32 kg/h Katalysatorlösung mit 36,6 Gew% Acetanhydrid (117 g) und 41,9 Gew% Essigsäure (134 g) gewonnen. Nach Abzug der Einsatzmengen betragen, bezogen auf den Acetaldehydumsatz, die Ausbeuten an Acetanhydrid 1002 g/h (9,82 Mol) = 72,2% und an Essigsäure 398 g/h (6,63 Mol) = 24,4%. Die Gesamtausbeute beläuft sich auf 96,6%. Die Raumleistung beträgt 1273 g Acetanhydrid + Essigsäure je Liter freies Reaktorvolumen und Stunde.

### Beispiel 5 (Vergleichsbeispiel)

Es wird unter den im Beispiel 1 angegebenen Bedingungen gearbeitet, wobei jedoch die Verweilzeit auf 20 min erhöht ist. Eingesetzt werden 2,15 kg/h Ausgangsmischung mit 40 Gew% Acetaldehyd (19,5 Mol), 17 Gew% Essigsäure (365 g) und 1,5 Gew% Acetanhydrid (32 g). Zur Einhaltung eines Sauerstoffspiegels von 2—5 Vol% im Reaktorabgas werden 240 l/h $O_2$ auf drei Stellen des Reaktors (4) verteilt zudosiert. 2,435 kg/h Reaktonsprodukt gelangen in die Destillationskolonne (13). Es werden 0,92 kg/h Esterphase mit 2,5 Gew% Wasser und 1,85 Gew% Acetaldehyd (17 g) sowie 0,11 kg/h wäßrige Phase erhalten. Daraus errechnet sich ein Acetaldehydumsatz von 98%. Bei einem Destillat-Sumpf-Gewichts-Verhältnis von 6 : 1 werden im Verdampfer (15) 1,205 kg/h Destillat mit 44,2 Gew% Acetanhydrid (533 g) und 55,8 Gew% Essigsäure (672 g) sowie 0,2 kg/h Katalysatorlösung mit 15,9 Gew% Acetanhydrid (32 g) und 68,5 Gew% Essigsäure (137 g) gewonnen. Nach Abzug der Einsatzmengen betragen, bezogen auf den Acetaldehydumsatz, die Ausbeuten an Acetanhydrid 533 g/h (5,23 Mol) = 54,7% und an Essigsäure 444 g/h (7,4 Mol) = 38,7%. Die Gesamtausbeute beläuft sich auf 93,4%. Die Raumleistung beträgt 888 g Acetanhydrid + Essigsäure je Liter freies Reaktorvolumen und Stunde.

Die Gegenüberstellung der erfindungsgemäßen Beispiele 1—3 zu den Vergleichsbeispielen 4 und 5 verdeutlicht den technischen Vorteil des erfindungsgemäßen Verfahrens bezüglich Acetaldehydumsatz, Acetanhydridausbeute und Raumleistung:

| Beispiel | T °C | Verweilzeit (min) | % Umsatz Acetaldehyd | % Ausbeute Acetanhydrid | Raumleistung g $Ac_2O$ + AcOH / l.h |
|---|---|---|---|---|---|
| 1 | 75 | 10 | 96,9 | 71,9 | 1741 |
| 2 | 75 | 7 | 96,0 | 75,8 | 2455 |
| 3 | 75 | 10 | 96,8 | 71,6 | 1731 |
| 4 | 50 | 10 | 70,1 | 72,2 | 1273 |
| 5 | 75 | 20 | 98 | 54,7 | 888 |

## Patenansprüche

1. Verfahren zur kontinuierlichen Herstellung von Essigsäure und Essigsäureanhydrid durch Oxydation von Acetaldehyd mit Säuerstoffgas in flüssiger Phase in Gegenwart von Kupferacylat und Kobaltacylat als Katalysator und in Gegenwart eines aliphatischen Carbonsäureesters als Verdünnungsmittel, dadurch gekennzeichnet, daß man die Umsetzung bei Temperaturen von 62 bis 90°C und Verweilzeiten von weniger als 20 min mit einem Gewichtsverhältnis des Verdünnungsmittels zu Acetaldehyd von 60:40 bis 40 : 60 durchführt, und daß man die Reaktionsprodukte einer Destillationszone zuführt, über Kopf Carbonsäureester und Wasser abdestilliert, in zwei Phasen trennt, die organische Phase unter einem Rückfluß von mindestens 1 : 1 zur Destillationszone zurückführt, das Sumpfprodukt der Destillationszone einer Verdampferzone zuleitet und dort 2 bis 12 Gewichtsteile eines Essigsäure/Essigsäureanhydrid-Gemisches als Destillat je 1 Gewichtsteil Katalysatorlösung als Sumpfprodukt gewinnt.

2. Verfahren nach Anspruch 1, dadurch gekennzeichnet, daß man die Umsetzung in einer langgestreckten, senkrechtstehenden Reaktionszone durchführt und die berechnete Menge Sauerstoffgas gleichzeitig an mehreren Stellen unterschiedlicher Höhe in die Reaktionszone eindosiert.

3. Verfahren nach einem der vorhergehenden Ansprüche, dadurch gekennzeichnet, daß man das Sauerstoffgas in einer solchen Menge in die Reaktionszone eindosiert, daß der Sauerstoffgehalt des Abgases geringer als 5 Vol% ist.

## Revendications

1. Procédé pour la préparation continue d'acide acétique et d'anhydride acétique par oxydation de l'acétaldéhyde par l'oxygène gazeux en phase liquide en présence d'acylate de cuivre et d'acylate de cobalt comme catalyseur et en présence d'un ester d'acide carboxylique aliphatique comme agent diluant, caractérisé en ce que l'on effectue la réaction à des températures de 62 à 90°C et avec des durées de passage de moins de 20 min, dans un rapport pondéral de l'agent diluant à l'acétaldéhyde de 60:40 à 40:60, et en ce que l'on envoie les produits de réaction à une zone de distillation, on distille en tête l'ester d'acide carboxylique et l'eau, on les sépare en deux phases, on renvoie la phase organique à la zone de distillation avec un reflux d'au moins 1:1, on envoie le produit de fond de la zone de distillation à une zone d'évaporation et on récupère de celle-ci 2 à 12 parties en poids d'un mélange acide acétique/anhydride acétique comme distillat pour chaque partie en poids de solution de catalyseur comme produit de fond.

2. Procédé selon la revendication 1, caractérisé en ce que l'on effectue la réaction dans une zone de réaction verticale allongée et on dose la quantité calculée d'oxygène gazeux simultanément en plusieurs points à différentes hauteurs dans la zone de réaction.

3. Procédé selon les revendications précédentes, caractérisé en ce que l'on dose l'oxygène gazeux dans la zone de réaction en quantité telle que la teneur en oxygène du gaz résiduaire soit inférieure à 5% en volume.

## Claims

1. Process for the continuous production of acetic acid and acetic anhydride by oxidizing acetaldehyde with oxygen gas in the liquid phase in the presence of copper acylate and cobalt acylate as catalyst and in the presence of an aliphatic carboxylic acid ester as diluent, which comprises effecting the reaction at temperatures of 62 to 90°C and sojourn times of less than 20 minutes, the diluent and acetaldehyde being used in a weight ratio of 60 : 40 to 40 : 60, introducing the reaction products into a distillation zone, distilling off overhead carboxylic acid ester and water separating the material distilled off overhead into two phases, recycling the organic phase to the distillation zone under a reflux of at least 1 : 1, delivering product accumulating in the base portion of the distillation zone to an evaporator zone and producing therein, as distillate, 2 to 12 parts by weight of an acetic acid/acetic anhydride mixture per part by weight of catalyst solution as base product.

2. Process as claimed in claim 1, wherein the reaction is carried out in an elongate, reaction zone in upright position and the calculated quantity of oxygen gas is metered into the reaction zone simultaneously at different levels.

3. Process as claimed in any of the preceding claims, wherein the oxygen gas is metered into the reaction zone in a quantity necessary for the issuing gas to contain less than 5% by volume of oxygen.